# EUROPEAN PATENT SPECIFICATION

(11) **EP 2 945 626 B1**
(45) Date of publication and mention of the grant of the patent: **12.09.2018**
(21) Application number: 13705591.9
(22) Date of filing: 15.01.2013
(51) Int. Cl.: A61K 31/439, A61K 31/46, A61P 25/20

(54) **USE OF ALPHA 7 NICOTINIC RECEPTOR AGONISTS FOR THE TREATMENT OF NARCOLEPSY**
VERWENDUNG VON ALPHA-7-NIKOTINREZEPTOR-AGONISTEN ZUR BEHANDLUNG VON NARKOLEPSIE
UTILISATION D'AGONISTES DU RÉCEPTEUR NICOTINIQUE ALPHA 7 POUR LE TRAITEMENT DE LA NARCOLEPSIE

(43) Date of publication of application: 25.11.2015
(73) Proprietor: Novartis AG, 4056 Basel (CH)
(72) Inventor: FENDT, Markus, CH-4002 Basell (CH); FEUERBACH, Dominik, CH-4002 Basel (CH); GOMEZ-MANCILLA, Baltazar, CH-4002 Basel (CH); LOPEZ-LOPEZ, Cristina, CH-4002 Basel (CH); MCALLISTER, Kevin Hall, CH-4002 Basel (CH)
(74) Representative: Appleyard Lees IP LLP
(86) International application number: PCT/IB2013/050368
(87) International publication number: WO 2014/111751

(56) References cited:
- WO-A1-01/85727
- WO-A1-2004/016608
- WO-A1-2004/022556
- WO-A1-2004/029050
- WO-A1-2005/123732
- WO-A1-2006/005608
- WO-A1-2007/045478
- WO-A1-2007/068475
- WO-A1-2009/018505
- WO-A1-2010/085724
- WO-A2-2011/009890
- ARIAS HUGO R ET AL: "Different interaction between the agonist JN403 and the competitive antagonist methyllycaconitine with the human alpha7 nicotinic acetylcholine receptor.", BIOCHEMISTRY 18 MAY 2010, vol. 49, no. 19, 18 May 2010 (2010-05-18), pages 4169-4180, XP002694125, ISSN: 1520-4995
- FEUERBACH D ET AL: "The selective nicotinic acetylcholine receptor alpha7 agonist JN403 is active in animal models of cognition, sensory gating, epilepsy and pain", NEUROPHARMACOLOGY, PERGAMON PRESS, OXFORD, GB, vol. 56, no. 1, 1 January 2009 (2009-01-01), pages 254-263, XP025846225, ISSN: 0028-3908, DOI: 10.1016/J.NEUROPHARM.2008.08.025 [retrieved on 2008-08-28]
- LAMMERS GERT JAN ET AL: "Pharmacological management of narcolepsy", EXPERT OPINION ON PHARMACOTHERAPY, ASHLEY PUBLICATIONS LTD, LONDON, UK, vol. 4, no. 10, 1 October 2003 (2003-10-01), pages 1739-1746, XP009151585, ISSN: 1465-6566
- HOUGHTON W C ET AL: "Pharmacotherapy for cataplexy", SLEEP MEDICINE REVIEWS, W.B. SAUNDERS, AMSTERDAM, NL, vol. 8, no. 5, 1 October 2004 (2004-10-01) , pages 355-366, XP004539475, ISSN: 1087-0792, DOI: 10.1016/J.SMRV.2004.01.004
- BANERJEE D ET AL: "Pharmacotherapy for excessive daytime sleepiness", SLEEP MEDICINE REVIEWS, W.B. SAUNDERS, AMSTERDAM, NL, vol. 8, no. 5, 1 October 2004 (2004-10-01) , pages 339-354, XP004539474, ISSN: 1087-0792, DOI: 10.1016/J.SMRV.2004.03.002
- HAUSER ET AL.: "TC-5619: an alpha7 neuronal nicotinic receptor-selective agonist that demonstrates efficacy in animal models of the positive and negative symptoms and cognitive dysfunction of schizophrenia.", BIOCHEM PHARMACOL, vol. 78, no. 7, 2009, pages 803-812,
- L. DANE: "Targacept to discontinue development of TC-5619 for ADHD, eliminate jobs", FIRST WORD PHARMA, 17 September 2012 (2012-09-17),
- S. BOYCE ET AL.: "Analgesic and toxic effects of ABT-594 resemble epibatidine and nicotine in rats.", PAIN, vol. 85, no. 3, April 2000 (2000-04), pages 443-450,
- G. M. CRAGG, D. J. NEWMAN: "Comprehensive medicinal chemistry II. 1.08.7.3 The Epibatidines and Cytisine", SCIENCE DIRECT, TEBANICLINE, AN OVERVIEW, GLOBAL PERSPECTIVE, 2007, pages 255-403,

## Description

The present invention relates to pharmaceutical uses of alpha 7 nicotinic acetylcholine receptor (α7 nAChR) agonists.

Narcolepsy is a chronic neurological sleep disorder caused by the brain's inability to regulate sleep-wake cycles normally. At various times throughout the day, people with narcolepsy experience irresistible bouts of sleep. If the urge becomes overwhelming, individuals will fall asleep for periods lasting from a few seconds to several minutes. There are three forms of narcolepsy: narcolepsy with cataplexy; narcolepsy without cataplexy; and narcolepsy due to medical condition (NDMC).

Common symptoms of narcolepsy are: excessive daytime sleepiness (EDS); cataplexy; abnormal REM sleep; nocturnal sleep disruption; paralysis during sleep onset or during awakening; and/or hypnagogic hallucinations.

Furthermore, EDS; cataplexy, a sudden loss of muscle tone most commonly in response to the sudden onset of strong emotions; and/or nocturnal sleep disruption, which is fragmenting the sleep during the night, are frequently affecting patients suffering from various disorders as diverse as sleep disorders, tumors, atypical depression, head trauma, anemia, kidney failure, hypothyroidism or an injury to the central nervous system. Besides narcolepsy, sleep disorders associated with EDS, cataplexy and/or nocturnal sleep disruption include sleep apnea, hypersomnia, circardian rhythm based disorders, restless legs syndrome and Niemann-Pick Disease. Particularly EDS is observed in most of these disorders. For review see e.g. Billiard M and EFNS Task Force; EFNS guidelines on management of narcolepsy, Eur J Neurol, (2006) Vol. 13 (10), pp. 1035-48.

The current treatments for narcolepsy are purely symptomatic and include (i) medications against sleepiness: e.g. amphetamine-like stimulants (e.g. Modafinil); and (ii) medications against other symptoms of narcolepsy (e.g. abnormal REM sleep, cataplexy, paralysis during sleep onset or during awakening, hypnagogic hallucinations): e.g. antidepressants and sodium oxybate. These treatments induce numerous side effects as well as residual sleepiness and cataplexy episodes. These treatments also disturb nocturnal sleep in many patients and tolerance may develop as a result of continuous treatment. Main issues with current treatments are: safety (antidepressants), efficacy (modafinil) and drug abuse potential (amphetamine-like, sodium oxybate). For review see e.g. Seiji Nishino, J Clin Psychiatry, 2007; 68 suppl. 13; Seiji Nishino et al. Sleep Medicine Reviews, Vol. 4, No. 1, pp 57-99, 2000 and Aldrich, M.S., 1998, Neurology 50: S2-S7.

Lammers and Overeem (Expert Opinion on Pharmacotherapy, Ashley Publications Ltd, London, UK, vol. 4, no. 10, 1 Oct 2003, pages 1739-1746) have reviewed the pharmacological management of narcolepsy.

Houghton, Scammell and Thorpy (Sleep Medicine Reviews (2004), 8, 355-366) have reviewed the safety and efficacy of major drug classes used to improve cataplexy in patients with narcolepsy.

Banerjee, Vitiello and Grunstein (Sleep Medicine Reviews (2004), 8, 339-354) have considered pharmacotherapy for excessive daytime sleepiness.

Consequently, there is a need for new pharmacological compounds that effectively target narcolepsy, EDS, nocturnal sleep disruption and/or cataplexy, but do not produce the adverse side-effects common in known treatment regiments.

It has been found that certain α7 nAChR agonists may be used in the treatment (whether therapeutic or prophylactic), prevention, amelioration or delay of progression of narcolepsy, EDS, nocturnal sleep disruption or cataplexy.

In particular it has been found that said α7 nAChR agonists may be used in the treatment, prevention, amelioration or delay of progression of narcolepsy.

In particular it has been found that said α7 nAChR agonists may be used in the treatment, prevention, amelioration or delay of progression of EDS, nocturnal sleep disruption or cataplexy associated with a sleep disorder, a tumor, an atypical depression, a head trauma, an anemia, a kidney failure, a hypothyroidism or an injury to the central nervous system. More particular it has been found that said α7 nAChR agonists may be used in the treatment, prevention, amelioration or delay of progression of EDS, nocturnal sleep disruption or cataplexy associated with a sleep disorder selected from the group consisting of narcolepsy, sleep apnea, hypersomnia, circadian rhythm based disorder, restless leg syndrome and Niemann-Pick Disease.

More particular it has been found that said α7 nAChR agonists may be used in the treatment, prevention, amelioration or delay of progression of EDS, nocturnal sleep disruption or cataplexy associated with a sleep disorder selected from the group consisting of sleep apnea, hypersomnia, circadian rhythm based disorder, restless leg syndrome and Niemann-Pick Disease.

Accordingly, a first aspect of the invention concerns an α7 nAChR agonist for use in the treatment, amelioration, prevention or delay of progression of narcolepsy, EDS, nocturnal sleep disruption or cataplexy;
wherein said α7 nAChR agonist is
(R)-3-(6-p-tolyl-pyridin-3-yloxy)-1-aza-bicyclo[2.2.2]octaneH-indole;
in free base form or in acid addition salt form.

In particular the invention concerns the use of the α7 nAChR agonist of the invention for the treatment, amelioration, prevention or delay of progression of narcolepsy.

In particular the invention concerns the use of the α7 nAChR agonist of the invention for the treatment, amelioration, prevention or delay of progression of EDS.

In particular the invention concerns the use of the α7 nAChR agonist of the invention for the treatment, amelioration, prevention or delay of progression of nocturnal sleep disruption.

In particular the invention concerns the use of the α7 nAChR agonist of the invention for the treatment, amelioration, prevention or delay of progression of cataplexy.

In particular the invention concerns the use of the α7 nAChR agonist of the invention for the treatment, amelioration, prevention or delay of progression of EDS, nocturnal sleep disruption or cataplexy associated with a sleep disorder, a tumor, an atypical depression, a head trauma, an anemia, a kidney failure, a hypothyroidism or an injury to the central nervous system.

More particularly the invention concerns the use of the α7 nAChR agonist of the invention for the treatment, amelioration, prevention or delay of progression of EDS, nocturnal sleep disruption or cataplexy associated with a sleep disorder selected from the group consisting of narcolepsy, sleep apnea, hypersomnia, circadian rhythm based disorder, restless leg syndrome and Niemann-Pick Disease.

More particularly the invention concerns the use of the α7 nAChR agonist of the invention for the treatment, amelioration, prevention or delay of progression of EDS, nocturnal sleep disruption or cataplexy associated with a sleep disorder selected from the group consisting of sleep apnea, hypersomnia, circadian rhythm based disorder, restless leg syndrome and Niemann-Pick Disease.

A further aspect of the invention relates to a method for the treatment, amelioration, prevention or delay of progression of narcolepsy, EDS, nocturnal sleep disruption or cataplexy in a subject in need of such treatment, which comprises administering to said subject a therapeutically effective amount of the α7 nAChR agonist of the invention.

In particular the invention relates to a method for the treatment, amelioration, prevention or delay of progression of narcolepsy in a subject in need of such treatment, which comprises administering to said subject a therapeutically effective amount of the α7 nAChR agonist of the invention.

In particular the invention relates to a method for the treatment, amelioration, prevention or delay of progression of EDS in a subject in need of such treatment, which comprises administering to said subject a therapeutically effective amount of the α7 nAChR agonist of the invention.

In particular the invention relates to a method for the treatment, amelioration, prevention or delay of progression of nocturnal sleep disruption in a subject in need of such treatment, which comprises administering to said subject a therapeutically effective amount of the α7 nAChR agonist of the invention.

In particular the invention relates to a method for the treatment, amelioration, prevention or delay of progression of cataplexy in a subject in need of such treatment, which comprises administering to said subject a therapeutically effective amount of the α7 nAChR agonist of the invention.

In particular the invention concerns a method for the treatment, amelioration, prevention or delay of progression of EDS, nocturnal sleep disruption or cataplexy associated with a sleep disorder, a tumor, an atypical depression, a head trauma, an anemia, a kidney failure, a hypothyroidism or an injury to the central nervous system, in a subject in need of such treatment, which comprises administering to said subject a therapeutically effective amount of the α7 nAChR agonist of the invention

More particularly the invention concerns a method for the treatment, amelioration, prevention or delay of progression EDS, nocturnal sleep disruption or cataplexy associated with a sleep disorder selected from the group consisting of narcolepsy, sleep apnea, hypersomnia, circadian rhythm based disorder, restless leg syndrome and Niemann-Pick Disease in a subject in need of such treatment, which comprises administering to said subject a therapeutically effective amount of the α7 nAChR agonist of the invention.

More particularly the invention concerns a method for the treatment, amelioration, prevention or delay of progression EDS, nocturnal sleep disruption or cataplexy associated with a sleep disorder selected from the group consisting of sleep apnea, hypersomnia, circadian rhythm based disorder, restless leg syndrome and Niemann-Pick Disease in a subject in need of such treatment, which comprises administering to said subject a therapeutically effective amount of the α7 nAChR agonist of the invention.

A further aspect of the invention relates to the use of the α7 nAChR agonist of the invention for the manufacture of a medicament for the treatment, amelioration, prevention or delay of progression of narcolepsy, EDS, nocturnal sleep disruption or cataplexy.

In particular the invention relates to the use of the α7 nAChR agonist of the invention for the manufacture of a medicament for the treatment, amelioration, prevention or delay of progression of narcolepsy.

In particular the invention relates to the use of the α7 nAChR agonist of the invention for the manufacture of a medicament for the treatment, amelioration, prevention or delay of progression of EDS.

In particular the invention relates to the use of the α7 nAChR agonist of the invention for the manufacture of a medicament for the treatment, amelioration, prevention or delay of progression of nocturnal sleep disruption.

In particular the invention relates to the use of the α7 nAChR agonist of the invention for the manufacture of a medicament for the treatment, amelioration, prevention or delay of progression of cataplexy.

In particular the invention relates to the use of the α7 nAChR agonist of the invention for the manufacture of a medicament for the treatment, amelioration, prevention or delay of progression of of EDS, nocturnal sleep disruption or cataplexy associated with a sleep disorder, a tumor, an atypical depression, a head trauma, an anemia, a kidney failure, a hypothyroidism or an injury to the central nervous system.

More particularly the invention relates to the use of the α7 nAChR agonist of the invention for the manufacture of a medicament for the treatment, amelioration, prevention or delay of progression of EDS, nocturnal sleep disruption or cataplexy associated with a sleep disorder selected from the group consisting of narcolepsy, sleep apnea, hypersomnia, circadian rhythm based disorder, restless leg syndrome and Niemann-Pick Disease.

More particularly the invention relates to the use of the α7 nAChR agonist of the invention for the manufacture of a medicament for the treatment, amelioration, prevention or delay of progression of EDS, nocturnal sleep disruption or cataplexy associated with a sleep disorder selected from the group consisting of sleep apnea, hypersomnia, circadian rhythm based disorder, restless leg syndrome and Niemann-Pick Disease.

### α7 nAChR agonist of the invention:

As used herein an "α7 nAChR agonist" is a compound that binds to a receptor comprising an α7 nAChR subunit in vivo and in vitro and is activating the receptor. Activation can be measured by the method disclosed in WO2001/85727, i.e. a functional affinity assay at the homomeric α7 nAChR carried out with a rat pituitary cell line stably expressing the α7 nAChR. As read out, the calcium influx upon stimulation of the receptor compared to epibatidine is used. "α7 nAChR agonists of the invention" according to the invention typically induce calcium influx of at least 50% of the maximal influx evoked by epibatidine with an EC₅₀ value of at least 1µM.

In one embodiment, the α7 nAChR agonist of the invention is selective for a receptor comprising a nicotinic acetylcholine receptor alpha 7 subunit, since such an agonist would be expected to cause fewer side effects than a non-selective agonist to a treated subject. An agonist being selective for a receptor comprising a nicotinic acetylcholine receptor alpha 7 subunit has a functional affinity to such a receptor to a much higher degree, e.g. at least 10-fold affinity difference in EC₅₀ value, preferably at least 20-fold, more preferably at least 50-fold, compared to any other nicotinic acetylcholine receptor. To assess the affinity of the α7 nAChR agonists of the invention on other nicotinic acetylcholine receptors, the method disclosed in WO2001/85727 can be used, i.e. to assess the affinity on human neuronal α 4β2 nAChR, a similar functional assay is carried out using a human embryonic kidney cell line stable expressing the human α4β2 subtype and to assess the activity of the compounds of the invention on the "ganglionic subtype" and the "muscle type" of nicotinic acetylcholine receptor, similar functional assays are carried out with a human embryonic kidney cell line stably expressing the human "ganglionic subtype" or a cell line endogenously expressing the human "muscle type" of nicotinic acetylcholine receptors.

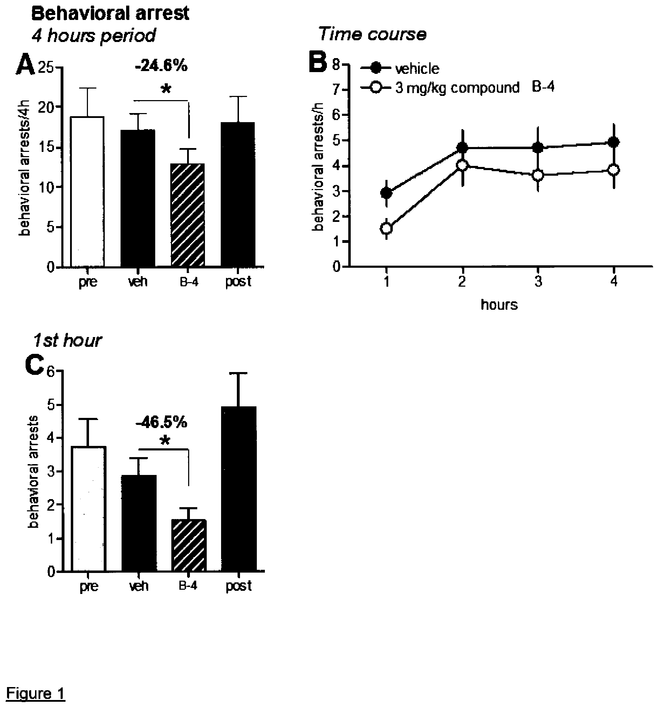

In the last 15 years much effort has been focused on developing selective α7 nAChR agonists leading to the discovery of many different chemotypes displaying said selective activity. These efforts are summarized the review from Horenstein et al (Mol Pharmacol, 2008, 74, 1496-1511, which describes no less than 9 different families of α7 nAChR agonists, in most of which selective agonists have been found. All compounds disclosed in figure 1 of said review are incorporated herein by reference. In fact, several drug candidates having an α7 nAChR agonist mode of action entered pre-clinical or even clinical testing (for review: Broad et al, Drugs of the Future, 2007, 32(2), 161-170; Romanelli et al, Expert Opin Ther Patents, 2007, 17(11), 1365-1377). Examples of such compounds - again belonging to a diversity of chemotypes - are MEM3454, MEM63908, SSR180711, GTS21, EVP6124, ABT107 and TC-5619. Further α7 nAChR agonists and their use as pharmaceuticals are known, for example, from WO2001/85727, WO2004/022556, WO2005/118535, WO2005/123732, WO2006/005608, WO2007/045478, WO2007/068476 and WO2007/068475.

The acid addition salt forms of the α7 nAChR agonists of the invention are preferably pharmaceutically acceptable salt forms. Such salts are known in the field (e.g. S.M. Berge, et al, "Pharmaceutical Salts", J. Pharm. Sd., 1977, 66:1-19; and "Handbook of Pharmaceutical Salts, Properties, Selection, and Use", Stahl, RH., Wermuth, C.G., Eds.; Wiley-VCH and VHCA: Zurich, 2002). A "pharmaceutically acceptable salt form" is intended to mean a salt form that is not toxic, biologically intolerable, or otherwise biologically undesirable.

The α7 nAChR agonist of the invention is B-1: (R)-3-(6-p-tolyl-pyridin-3-yloxy)-1-aza-bicyclo[2.2.2]octane.

The α7 nAChR agonist of the invention is a compound B-1 in free base form or in acid addition salt form.

The compound and its manufacture are known from WO2004/022556, or can be prepared analogously to said reference.

### Disorders/Symptoms/Therapeutic Methods:

Narcolepsy is a neurological sleep disorder which can be subdivided into three forms: narcolepsy with cataplexy, narcolepsy without cataplexy and narcolepsy due to medical condition (NDMC).

Narcolepsy often has the following symptoms: excessive daytime sleepiness (EDS); cataplexy (in narcolepsy with cataplexy); abnormal REM sleep; nocturnal sleep disruption; paralysis during sleep onset or during awakening; and/or hypnagogic hallucinations.

Narcolepsy Due to Medical Condition (NDMC) is a group of disorders also known as secondary or symptomatic narcolepsy. Medical conditions commonly causing narcolepsy with cataplexy may be: tumors, sarcoidosis, arteriovenous malformations affecting the hypothalamus, multiple sclerosis plaques impairing the hypothalamus, paraneoplastic syndrome anti-Ma2 antibodies, Neimann-Pick type C disease or Coffin-Lowry syndrome. Medical condition commonly causing narcolepsy without cataplexy may be: head trauma, myotonic dystrophy, Prader-Willi syndrome, Parkinson's disease or multisystem atrophy.

The term "paralysis during sleep onset or during awakening" as used herein, is the temporary inability to talk and/or move during sleep onset or during awakening. Sleep paralysis may last from a few seconds to minutes.

The term "hypnagogic hallucinations" as used herein, refers to dream-like auditory or visual hallucinations during sleep onset or during awakening. Hypnagogic hallucinations can be vivid, often frightening, dreamlike experiences that occur at sleep onset or during awakening.

The characteristics of excessive daytime sleepiness (EDS) include e.g. repeated episodes of naps or lapses into sleep of short duration, usually less than an hour. The patient e.g. sleeps for 10 to 20 minutes and awakens refreshed but begins to feel sleepy again and the pattern repeats itself. In patients afflicted with EDS sleep usually occurs in situations in which tiredness is common, such as traveling in transport; attending a monotonous meeting that requires no active participation; or listening to a play, concert, movie or lecture, but there may also be sudden and irresistible sleep attacks in situations where sleep normally never occurs, including during an examination, at interactive business talks, while eating, walking or driving and when actively conversing. Usually sleep attacks occur on a background of drowsiness that is a common daily feature.

The term "excessive daytime sleepiness" as used herein, unless otherwise indicated, means any EDS which accompanies, or follows in the course of, or which is caused by e.g. a sleep disorder, a tumor, an atypical depression, a head trauma, an anemia, a kidney failure, a hypothyroidism or an injury to the central nervous system. In one embodiment, said sleep disorder is selected from the group consisting of narcolepsy, sleep apnea, hypersomnia, circadian rhythm based disorder, restless leg syndrome and Niemann-Pick Disease. In one embodiment, said sleep disorder is selected from the group consisting of sleep apnea, hypersomnia, circadian rhythm based disorder, restless leg syndrome and Niemann-Pick Disease.

The term "nocturnal sleep disruption" as used herein, unless otherwise indicated, means any disruption of normal night sleep patterns which accompanies, or follows in the course of, or which is caused by e.g. a sleep disorder, a tumor, an atypical depression, a head trauma, an anemia, a kidney failure, a hypothyroidism or an injury to the central nervous system. In one embodiment, said sleep disorder is selected from the group consisting of narcolepsy, sleep apnea, hypersomnia, circadian rhythm based disorder, restless leg syndrome and Niemann-Pick Disease. In one embodiment, said sleep disorder is selected from the group consisting of sleep apnea, hypersomnia, circadian rhythm based disorder, restless leg syndrome and Niemann-Pick Disease.

Cataplexy is characterized by sudden loss of muscle tone. The duration of cataplexy is usually short, ranging from a few seconds to several minutes and recovery is immediate and complete. The loss of muscle tone varies in severity and ranges from a mild sensation of weakness with head droop, facial sagging, jaw drop, slurred speech and buckling of the knees to complete postural collapse, with a fall to the ground. Cataplexy is usually precipitated by emotion that usually has a pleasant or exciting component, such as laughter, elation, pride, anger or surprise. Cataplexy may be associated with narcolepsy. Cataplexy may be associated with specific lesions located primarily in the lateral and posterior hypothalamus, as e.g tumors (astrocytoma, glioblastoma, glioma, craniopharyngioma and subependynoma) and arterio-venous malformations. Conditions in which cataplexy can be seen include ischemic events, multiple sclerosis, head injury, paraneoplastic syndromes, and infections, such as encephalitis. Cataplexy may occur transiently or permanently due to lesions of the hypothalamus that were caused by surgery, especially in difficult tumor resections. In infancy cataplexy can been seen in association with other neurological syndromes such as Niemann-Pick type C disease. For review see e.g. Mignot, E., W. Chen, and J. Black, On the value of measuring CSF hypocretin-1 in diagnosing narcolepsy. Sleep 2003. 26(6): p. 646-9; Einhaus SI, S.R., Craniopharyngioma, in Principles and Practice of Pediatric Neurosurgery, P.I. Albright AL, Adelson PD, Editor. 1999, Thieme: New York. p. 545-562; Malik, S., et al., Narcolepsy associated with other central nervous system disorders; Neurology, 2001. 57(3): p. 539-41; Marcus, C.L., et al., Secondary narcolepsy in children with brain tumors; Sleep, 2002. 25(4): p. 435-9; Vankova, J., et al., Sleep disturbances and hypocretin deficiency in Niemann-Pick disease type C. Sleep, 2003. 26(4): p. 427.

The term "cataplexy" as used herein, unless otherwise indicated, means any event of cataplexy which accompanies, or follows in the course of, or which is caused by e.g. a sleep disorder, a tumor, an atypical depression, a head trauma, an anemia, a kidney failure, a hypothyroidism or an injury to the central nervous system. In one embodiment, said sleep disorder is selected from the group consisting of narcolepsy, sleep apnea, hypersomnia, circadian rhythm based disorder, restless leg syndrome and Niemann-Pick Disease. In one embodiment, said sleep disorder is selected from the group consisting of sleep apnea, hypersomnia, circadian rhythm based disorder, restless leg syndrome and Niemann-Pick Disease.

Within the present invention, an atypical depression may be, but is not limited to, a subtype of dysthymia and major depression characterized by mood reactivity and by reversed vegetative symptoms, namely over-eating and over-sleeping as described by The Diagnostic and Statistical Manual of Mental Disorders (DSM-IV-TR) and The International Statistical Classification of Diseases and Related Health Problems 10th Revision (ICD-10)

Sleep disorders associated with EDS, cataplexy and/or nocturnal sleep disruption may be e.g.:
Narcolepsy: as described above.

Sleep apnea: sleep apnea is characterized by a cessation or decrease of ventilatory effort during sleep and is usually associated with oxygen desaturation. This disorder is usually associated with a complaint of insomnia with an inability to maintain sleep, however excessive sleepiness can also occur. Feelings of daytime tiredness/sleepiness are common. Within the present invention sleep apnea may be but is not limited to central sleep apnea syndrome as described in the Standards of the International Classification of Sleep Disorders revised, produced by the American Academy of Sleep Science ISBN 0-9657220-1-5.

Hypersomnia: Within the present invention hypersomnia may be, but is not limited to, disorders of excessive somnolence as described in the Standards of the International Classification of Sleep Disorders revised, produced by the American Academy of Sleep Science ISBN 0-9657220-1-5.

Circadian Rhythm Based Disorder: Within the present invention, a circadian rhythm based disorder may be, but is not limited to, time zone change (jet lag) syndrome, shift work sleep disorder, irregular sleep-wake pattern, delayed sleep-phase syndrome, advanced sleep-phase syndrome, non-24-hour sleep-wake disorder and circadian rhythm sleep disorder not otherwise specified, as described in the Standards of the International Classification of Sleep Disorders revised, produced by the American Academy of Sleep Science ISBN 0-9657220-1-5.

Restless Legs Syndrome: Within the present invention restless legs syndrome may be, but is not limited to, restless legs syndrome as characterized by a disagreeable leg sensation that usually occurs prior to sleep onset and that causes an almost irresistible urge to move the legs, as described in the Standards of the International Classification of Sleep Disorders revised, produced by the American Academy of Sleep Science ISBN 0-9657220-1-5. Niemann-Pick Disease: Within the present invention Niemann-Pick Disease may be, but is not limited to, Niemann-Pick Disease referring to a group of fatal inherited metabolic disorders that are included in the larger family of lysosomal storage diseases (LSDs) resulting usually hepatosplenomegaly, lymphadenopathy, anemia and mental and physical deterioration. For reference see e.g. International Statistical Classification of Diseases and Related Health Problems 10th Revision (ICD-10) and in the following publication: Winchester B, Vellodi A, Young E (2000) "The molecular basis of lysosomal storage diseases and their treatment". Biochem. Soc. Trans. 28 (2): 150-4.

The term "subject" as used herein refers to a human being, especially to a patient suffering from narcolepsy, EDS, nocturnal sleep disruption and/or cataplexy.

The term "treatment" as used herein refers to any type of treatment that imparts a benefit to a subject, especially a patient, suffering from narcolepsy, EDS, nocturnal sleep disruption and/or cataplexy, including a reduction of one or more characteristics associated with narcolepsy, EDS, nocturnal sleep disruption and/or cataplexy or prevention/delay of the onset/progression of narcolepsy, EDS, nocturnal sleep disruption and/or cataplexy (e.g. prophylactic treatment).

The term "therapeutically effective amount" as used herein typically refers to a drug amount which, when administered to a subject, is sufficient to provide a therapeutic benefit, e.g. is sufficient for treating, ameliorating, preventing or delaying the progression of narcolepsy, EDS, nocturnal sleep disruption and/or cataplexy (e.g. the amount provides a amelioration of the characteristics associated with narcolepsy, EDS, nocturnal sleep disruption and/or cataplexy, e.g. it leads to an increased period of time between episodes of cataplexy).

Treatment may comprise a reduction in the characteristics associated with narcolepsy, EDS, nocturnal sleep disruption and/or cataplexy, including for example, although not limited to, a reduction in the degree of sleepiness or drowsiness; a reduction in the number of involuntary sleep attacks; an improvement in the ability to preserve the sleepiness or drowsiness; an improvement of night time sleep consolidation; an improvement in the well-being of the subject; an improvement in the ability to carry out normal tasks; an improved ability to drive cars and operate machines, and/or an increased period of time between sleep attacks and/or episodes of cataplexy.

One aspect of the treatment of narcolepsy, EDS, nocturnal sleep disruption and/or cataplexy, is that said treatment should have a minimal adverse effect on the patient, e.g. the drug used should have a high level of cardiac safety. Highly relevant would be e.g. an agent that can be used to treat narcolepsy, EDS, nocturnal sleep disruption and/or cataplexy without producing the side effects of the known treatment regiments. Further highly relevant for the treatment of (i) narcolepsy or (ii) EDS, nocturnal sleep disruption and/or cataplexy caused by a sleep disorder sleep disorder would be an agent that has a positive effect on the treatment of narcolepsy or the sleep disorder itself.

In the case of prophylactic treatment, the α7 nAChR agonists of the invention may be used to delay or prevent the onset of e.g. narcolepsy, EDS, nocturnal sleep disruption and/or cataplexy, which in some cases may be acquired at later stages of life of a subject, e.g. during puberty.

For the above-mentioned treatment methods the appropriate dosage will vary depending upon, for example, the compound employed, the host, the mode of administration and the nature and severity of the condition/symptom being treated. However, in general, satisfactory results in animals are indicated to be obtained at a daily dosage of from 0.1 to 100 mg/kg body weight, preferably from 1 to 50 mg/kg body weight, e.g. 10 mg/kg. In larger mammals, for example humans, an indicated daily dosage is in the range from 0.5 to 500 mg, preferably from 1 to 100 mg, most preferably from 2 to 75 mg, e.g. 25 mg or 50 mg of an α7 nAChR agonist of the invention conveniently administered, for example, in divided doses up to four times a day.

In one embodiment, the daily dosage of the α7 nAChR agonist of the invention is from 1 to 100 mg.

In one embodiment, the daily dosage of the α7 nAChR agonist of the invention is from 2 to 75 mg.

In one embodiment, the daily dosage of the α7 nAChR agonist of the invention is about 25 mg.

In one embodiment, the daily dosage of the α7 nAChR agonist of the invention is about 50 mg.

### Pharmaceutical compositions:

For use according to the invention, the α7 nAChR agonist of the invention may be administered as single active agent or in combination with other active agents, in any usual manner, e.g. orally, for example in the form of tablets or capsules, parenterally, for example in the form of injection solutions or suspensions, or transdermally, for example in the form of a patch.

In one embodiment, the manner of administration is oral administration, for example in the form of tablets or capsules.

In one embodiment, the manner of administration is transdermal administration, for example in the form of a patch.

Moreover, the present invention provides a pharmaceutical composition comprising an α7 nAChR agonist of the invention in association with at least one pharmaceutical carrier or diluent for the treatment, amelioration, prevention or delay of progression narcolepsy, EDS, nocturnal sleep disruption and/or cataplexy. Such compositions may be manufactured in conventional manner. Unit dosage forms may contain, for example, from about 2.5 to about 25 mg of the α7 nAChR agonist of the invention.

The pharmaceutical compositions according to the invention are compositions for enteral, such as nasal, rectal or oral; parenteral, such as intramuscular or intravenous; or transdermal (e.g. by a patch) administration to warm-blooded animals (human beings and animals) that comprise an effective dose of the pharmacological active ingredient alone or together with a significant amount of a pharmaceutically acceptable carrier. The dose of the active ingredient depends on the species of warm-blooded animal, body weight, age and individual condition, individual pharmacokinetic data, the disease to be treated and the mode of administration.

The pharmaceutical compositions comprise from approximately 1% to approximately 95%, preferably from approximately 20% to approximately 90%, active ingredient. Pharmaceutical compositions according to the invention may be, for example, in unit dose form, such as in the form of ampoules, vials, suppositories, dragees, tablets or capsules.

The pharmaceutical compositions of the present invention are prepared in a manner known per se, for example by means of conventional dissolving, lyophilizing, mixing, granulating or confectioning processes. Such processes are exemplified in WO 2005/079802, WO 2003/047581, WO 2004/000316, WO 2005/044265, WO 2005/044266, WO 2005/044267, WO 2006/114262 and WO 2007/071358.

Compositions for transdermal are described in Remington's Pharmaceutical Sciences 16th Edition Mack; Sucker, Fuchs and Spieser, Pharmazeutische Technologie, 1st Edition, Springer.

The following are further embodiments of the invention.

The following non-limiting examples are illustrative of the disclosure.

### 1. Formulation Examples

### 1.1 Hard Capsules

Hard gelatin capsules, each comprising as active ingredient 0.5, 5 or 25 mg of the mono-fumarate of compound B-1, i.e. of (R)-3-(6-(4-methylphenyl)-pyridin-3-yloxy)-1-aza-bicyclo[2.2.2]octane, can be prepared as follows:

| Ingredient for capsule fill | % (w/w) for 0.5 mg capsules | % (w/w) for 5 mg capsules | % (w/w) for 25 mg capsules |
|---|---|---|---|
| Mono-fumarate of (R)-3-(6-(4-methylphenyl)-pyridin-3-yloxy)-1-aza-bicyclo[2.2.2]octane | 0.46 | 4.65 | 23.23 |
| Lactose monohydrate | 65.24 | 61.05 | 42.47 |
| Microcrystalline cellulose | 25.00 | 25.00 | 25.00 |
| Hypromellose | 2.50 | 2.50 | 2.50 |
| Sodium croscarmellose | 6.00 | 6.00 | 6.00 |
| Colloidal silicon dioxide | 0.30 | 0.30 | 0.30 |
| Magnesium stearate | 0.50 | 0.50 | 0.50 |
| Purified water* | q.s. | q.s. | q.s. |

| | | | |
|---|---|---|---|
| *removed during processing | | | |

Preparation process: Mono-fumarate of (R)-3-(6-(4-methylphenyl)-pyridin-3-yloxy)-1-aza-bicyclo[2.2.2]octane, lactose monohydrate, microcrystalline cellulose, a portion of sodium croscarmellose and hypromellose are dry mixed in a high shear mixer bowl, and granulating fluid (purified water) added. Once the granulation is complete, the wet granules are dried in a fluid bed drier and the dry granules are milled. The remaining sodium croscarmellose and colloidal silicon dioxide are passed through a suitable sieve and added to the dried granular material and blended in a suitable blending shell. This is achieved by co-sieving the sodium croscarmellose and the colloidal silicon dioxide with a portion of the milled granules through a suitable sieve into the blending shell. Similarly, the required amount of sieved magnesium stearate is added to the bulk granule and then mixed in the same blending shell. This final blend is encapsulated into capsules using automated equipment. Weigth ratio of capsule fill to empty capsule shells is 2 : 1.

### 1.2: Tablets

### Example 1.2.1: Film-coated tablet

Film-coated tablets containing e.g. 0.5 mg of the mono-fumarate of (R)-3-(6-(4-methylphenyl)-pyridin-3-yloxy)-1-aza-bicyclo[2.2.2]octane may be prepared as follows:

### Preparation of pre-mix:

Weigh-in mono-fumarate of (R)-3-(6-(4-methylphenyl)-pyridin-3-yloxy)-1-aza-bicyclo[2.2.2]octane (e.g. approx. 0.7%) and maize starch (e.g. approx. 13%), mix in a tumble blender (approx 100-300 rotations), pass through a sieve of approx. 0.25-1.0 mm mesh-size. Mix in a tumble blender (approx. 100-300 rotations).

### Preparation of final blend:

To above pre-mix add microcrystalline cellulose (e.g. approx. 25%), sprayed lactose (e.g. approx. 68%), sodium-carboxymethylcellulose XL (e.g. approx. 2%) and Aerosil (e.g. approx. 0.5%) and mix in a tumble blender (approx. 100-300 rotations). Pass this mixture through a sieve of approx. 0.5-1.0 mm mesh-size and mix again (approx. 100-300 rotations).

Add the sodium-stearyl-fumarate (e.g. approx. 1.5%) through a handsieve at approx. 0.5-1.0 mm mesh-size and mix in a tumble blender (approx. 30-150 rotations).

### Compression:

On a rotary press compress the above final blend to cores of approx. 100mg, using the dosage specific tooling (e.g. approx. 6mm, round, curved).

### Coating:

Prepare a suspension in water with basic coating premixes black, red, yellow and/or white. Coat the above obtained cores in a perforated coating pan, and dry.

### Example 1.2.2: Bilayer film-coated tablet

Bilayer film-coated tablets containing e.g. 2.5 mg of the mono-fumarate of (R)-3-(6-(4-methylphenyl)-pyridin-3-yloxy)-1-aza-bicyclo[2.2.2]octane may be prepared as follows:

### Final active blend:

Weigh-in mono-fumarate of (R)-3-(6-(4-methylphenyl)-pyridin-3-yloxy)-1-aza-bicyclo[2.2.2]octane coarse (e.g. approx. 15.5%), microcrystalline cellulose (e.g. approx. 25%), sprayed lactose (e.g. approx. 53%), sodium-carboxymethylcellulose XL (e.g. approx. 3%) and Aerosil (e.g. approx. 0.5%) and mix in a tumble blender (approx 100-300 rotations). Pass this mixture through a sieve of approx. 0.5-1.0 mm mesh-size and mix again (approx 100-300 rotations).

Add the Na-stearyl-fumarate (e.g. approx. 3%) through a handsieve at approx. 0.5-10mm and mix in a tumble blender (approx 30-150 rotations).

### Final placebo blend:

Weigh-in microcrystalline cellulose (e.g. approx. 26%), sprayed lactose (e.g. approx. 69%), sodium-carboxymethylcellulose XL (e.g. approx. 1.9%) and Aerosil (e.g. approx. 0.5%) and mix in a tumble blender (approx 100-300 rotations). Pass this mixture through a sieve of approx. 0.5-1.0 mm mesh-size and mix again (approx 100-300 rotations).

Add the sodium-stearyl-fumarate (e.g. approx. 3%) through a handsieve at approx. 0.5-1.0 mm and mix in a tumble blender (approx 30-150 rotations).

### Compression:

On a rotary press compress the above final blends to a bilayer tablet-core of approx. 100mg with one placebo layer (approx. 77.5mg) and one active layer (approx. 22.5mg), using the dosage specific tooling (e.g. approx. 6mm, round, curved).

### Coating:

Prepare a suspension in water with basic coating premixes black, red, yellow and/or white. Coat the above obtained cores in a perforated coating pan, and dry.

### Example 1.2.3: Film-coated tablet

Film-coated tablets containing e.g. 50 mg of the mono-fumarate of (R)-3-(6-(4-methylphenyl)-pyridin-3-yloxy)-1-aza-bicyclo[2.2.2]octane may be prepared as follows:

### Final blend:

Weigh-in mono-fumarate of (R)-3-(6-(4-methylphenyl)-pyridin-3-yloxy)-1-aza-bicyclo[2.2.2]octane coarse (e.g. approx. 15.5%), microcrystalline cellulose (e.g. approx. 25%), sprayed lactose (e.g. approx. 53%), sodium-carboxymethylcellulose XL (e.g. approx. 3%) and Aerosil (e.g. approx. 0.5%) and mix in a tumble blender (approx. 100-300 rotations). Pass this mixture through a sieve of approx. 0.5-1.0 mm mesh-size and mix again (approx. 100-300 rotations).

Add the sodium-stearyl-fumarate (e.g. approx. 3%) through a handsieve at approx. 0.5-10mm and mix in a tumble blender (approx. 30-150 rotations).

### Compression:

Compress the above final blend on a rotary press to cores, using the dosage specific tooling (e.g. approx. 15*5.9mm, round, curved).

### Coating:

Prepare a suspension in water with basic coating premixes black, red, yellow and/or white. Coat the above obtained cores in a perforated coating pan, and dry.

The usefulness of the α7 nAChR agonists of the invention in the treatment of narcolepsy, EDS, nocturnal sleep disruption and/or cataplexy can be confirmed in a range of standard tests including those indicated below.

### 2. Preclinical testing

### 2.1. In-vitro Tests: Selectivity of Compounds A-1 and B-1

Based on the activity/selectivity data shown below it is concluded that said compounds are selective agonists at the α7-nAChR.

Assays: To assess α7-nAChR activity, a functional assay was employed using GH3 cells that recombinantly expressed human α7-nAChR. 40000 cells per well were seeded 48 h prior to the experiment on black 96-well plates (Costar) and incubated at 37°C in a humidified atmosphere (5 % CO₂/95 % air). On the day of the experiment, medium was removed by flicking the plates and replaced with 0.1 ml growth medium containing 0.002 mM Fluo-4, (Molecular Probes) in the presence of 2.5 mM probenecid (Sigma). The cells were incubated at 37°C in a humidified atmosphere (5 % CO2/95 % air) for 1 h. Plates were flicked to remove excess of Fluo-4, washed twice with Hepes-buffered salt solution (HBSS, in mM: NaCl 130, KCI 5.4, CaCl₂ 2, MgSO₄ 0.8, NaH₂PO₄ 0.9, glucose 25, Hepes 20, pH 7.4; HBS) and refilled with 0.1 ml of HBS containing antagonist when appropriate. The incubation in the presence of the antagonist lasted 3-5 minutes. Plates were placed in the cell plate stage of a FLIPR device (fluorimetric imaging plate reader, Molecular Devices, Sunnyvale, CA, USA). After recording of the baseline (laser: excitation 488 nm at 1 W, CCD camera opening of 0.4 seconds) the agonists (0.05 ml) were added to the cell plate using the FLIPR 96-tip pipettor while simultaneously recording the fluorescence. Calcium kinetic data were normalized to the maximal fitted response induced by epibatidine, which is a full agonist at α7-nAChR. Four parameter Hill equations were fitted to the concentration-response. Values of Emax (maximal effect in % compared to the epibatidine response) and EC₅₀ (concentration producing half the maximal effect in µM) were derived from this fit. Assay described in: D Feuerbach et al, Neuropharmacology (2005), 48, 215-227.

To assess the activities of the compounds on the other receptors (i.e. α1β1γδ; α4β2; α3β4 and 5-HT₃), similar functional assays were carried out.

| | Compound B-1 | Compound A-1 |
|---|---|---|
| α7 EC₅₀ (nM) ± SEM, n | 39 ± 4.7, 18 | 100 ± 7.9,17 |
| α7 Eₘₐₓ± SEM, n | 73 ± 4.1%, 18 | 85 ± 5%, 17 |
| α1β1γδ IC₅₀ (nM) ± SEM, n | 10715 ± 1376, 6 | 159222 ± 4306, 6 |
| α4β2 IC₅₀ (nM) ± SEM, n | 3981 ± 770, 6 | 25119 ± 4794, 5 |
| α3β4 IC₅₀(nM₎± SEM, n | 5248 ± 609, 5 | 23097 ± 4123, 6 |
| 5-HT₃ IC₅₀(nM)± SEM, n | 19054 ± 3811, 6 | 22324 ± 5869, 2 |

Compound A-1 is (S)-(1-aza-bicylo[2.2.2]oct-3-yl)carbonic acid(S)-1-(2-fluro-phenyl)-ethyl ester.

### 2.2. In-vivo pharmacology

### 2.2.1 Orexin-deficiency model:

### a) Animals

Male transgenic mice with a knockout of the orexin peptide gene are used. Orexin-deficient mice express a phenotype with cataplexy events (observed as 'behavioral arrests') and a disrupted sleep pattern including fragmented sleep and sleepiness in their active period.

### b) Assessment of behavior

The animals are observed and videotaped via infrared cameras during the first hours of the lights-off period which is the active phase of nocturnal rodents like mice. At this time, orexin-deficient mice show the most 'behavioral arrests'. The number of these events can be furthermore increased by presenting a new environment. For this purpose, new saw dust, a running wheel, marbles and tunnels are placed in the test environment. The number of 'behavioral arrests' are offline counted by the experimenter for each hour of recording.

Behavioral arrests were defined by phases of total inactivity outside the next box, which last longer than 10 second, preceded and followed by robust locomotor activity (see also Scammell et al., 2009, Sleep 32(1): 111-116). Some of the animals have temporal skull EEG electrodes implanted which are connected to transmitters. The EEG is recorded via a receiver and stored on a PC for further analysis. EEG is used to assess and to quantify sleep behavior and vigilance stage.

### c) Protocol

A cross-over design is used because of the high inter- and intra-individual variability of the behaviors to be observed. Furthermore, to avoid habituation to the test environment, the animals are only tested once per week in the test environment. During the remaining days, the animals are in their homecage.

For each experimental session, 3-4 mice are put into the test environment 3 hours before lights-off. The first experimental session (week 1) is used to get a baseline (without treatment). The second and third sessions (weeks 2+3) are used for treatment (vehicle & compound, cross-over design, administration 5 minutes before lights-off). The fourth and last experimental session is again without treatment to evaluate a post-treatment baseline.

### d) Results

Figure 1 depicts the effects of 3 mg/kg compound B-4 ((2S,3R)-3-[6-(1H-indol-5-yl)-pyridazin-3-yloxy]-2-methyl-1-aza-bicyclo[2.2.2]octane), orally administered directly before lights-off, on the number of narcoleptic episodes (behavioral arrests) in orexin-deficient mice. Compound B-4 decreased the number of narcoleptic episodes during the whole 4 hours observation period by ca. 25 % (Paired t-test: t₁₄ = 2.48, p = 0.03; Figure 1A+B). The reduction was more pronounced during the first hour (ca. 47%; t₁₄ = 2.32, p = 0.04; Figure 1C).

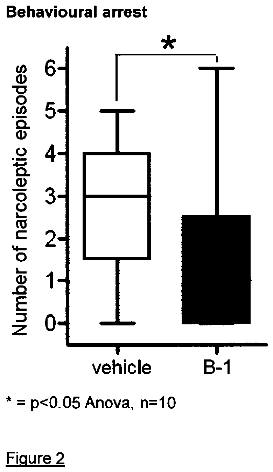

Figure 2 depicts the effects of 10 mg/kg compound B-1, orally administered directly before lights-off, on the number of narcoleptic episodes (behavioral arrests) in orexin-deficient mice. Compound B-1 significantly decreased the number of narcoleptic episodes during the first hour of the observation period by ca. 66 % (p<0.05 Anova).

### 2.2.2 EEG data

### a) Animals

Animals have temporal skull EEG electrodes implanted which are connected to transmitters.

Animals were group-housed (2 - 4) per cage at a constant temperature of 22 ± 1 °C, on a 12:12 light-dark schedule. The EEG is recorded via a receiver and stored on a PC for further analysis. EEG is used to assess and to quantify sleep behavior and vigilance stage.

### b) Protocol

Animals were given a 1-day period of acclimatization in the recording cages with recording cables connected. On the 1st day of the experiment, EEG recordings were made continuously during a 22-hours period beginning at 11 A.M., 15 min after vehicle administration. On the second day of the experiment, EEG recordings were carried out in the same way, but 15 minutes after drug administration. In this way, each animal served as its own control. 10 animals were recorded from simultaneously in each experiment.

Automatic evaluation of the nycthemeral cycle (REM sleep, classical sleep, wakefulness and an awake non-vigilant state termed "drowsiness") was performed as described (Vigouret et al., 1978 Pharmacology 16 Suppl 1:156-73). In the present context, "quiet wake" and "active wake", describe conscious animals in a resting and an active state, respectively, while "REM" and "NREM" represent sleep states.

### c) Results

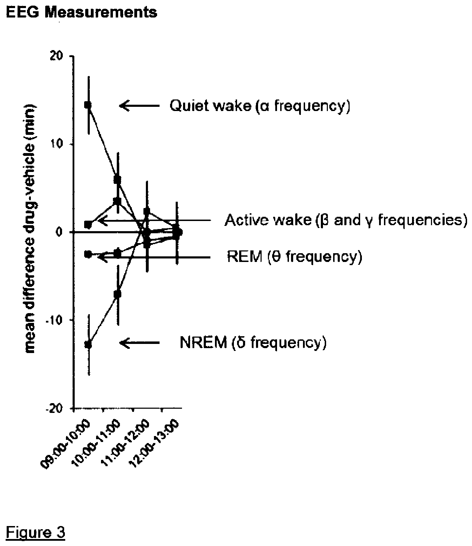

Figure 3 depicts the effects of 30 mg/kg compound B-1, orally administered 6 h after lights on (9:00) in mice (n=10) on quiet wake (α frequency, 9.5-12.5 Hz), active wake (β frequency, 12.5-30.5 Hz; and γ frequency, >30.5 Hz), REM sleep (θ frequency, 4.5-9.5 Hz) and NREM sleep (δ frequency, <4.5 Hz). Assessment period shown is 3 hours. Compound B-1 causes a decline of NREM sleep and increases the time spent in quiet wake. The numerical assessment of figure 3 is given in table 1.

**Table 1:**

| | Mean difference drug-vehicle (min) assessed at time point | | | |
|---|---|---|---|---|
| | 9:30 | 10:30 | 11:30 | 12:30 |
| Quiet wake (α frequency) | 14.3 | 6.1 | -1.8 | -0.9 |
| Active wake (β and γ frequencies) | 0.9 | 3.6 | 0.1 | 0.3 |
| REM (θ frequency) | -2.2 | -2.1 | -0.9 | -0.2 |
| NREM (δ frequency) | -12.4 | -7.1 | 2.1 | -0.1 |

### 2.2.3 telemethyl histamine

Histamine (HA) is released all over the brain by tuberomammillary nucleus neurons. Evidence indicates that histaminergic neurons have a major role in wakefulness control. HA is metabolized by histamine-N-methyltransferase to tele-methylhistamine (tMHA) in some tissues, including brain.

### a) Protocol

Mice (n=8) were treated during the first hour of the lights-on period and sacrificed 30 min later. Brain tissue was collected and subjected to LC-MS analysis (Croyal et al. Analytical Biochemistry, 2011, 409:28-36) to determine t-MHA levels.

### b) Results

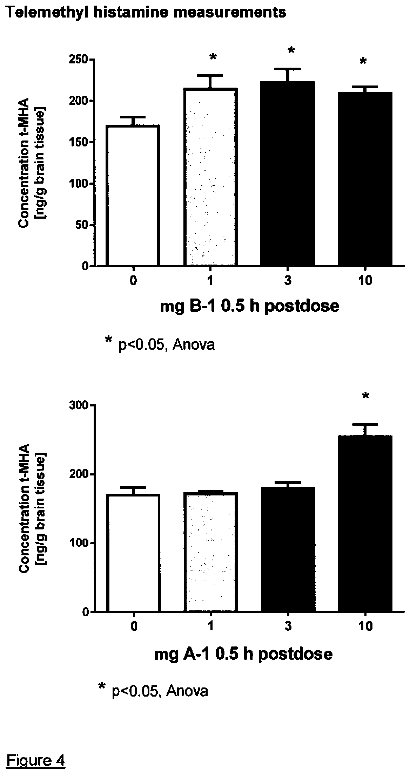

Figure 4 depicts the effects of different doses of compound B1 and A-1, orally administered to mice, 30 minutes after administration on brain tMHA levels. tMHA levels are significantly elevated for compound B-1 at all doses tested. For compound A-1, a significant elevation is seen for the 10 mg/kg dose.

### 3. Clinical Testing: Improvement Trials

Clinical testing of the α7 nAChR agonists of the invention may be conducted, for example, in one of the following study designs. The skilled physician may look at a number of aspects of patient behaviors and abilities. He will realize that such studies are considered as guidelines and the certain aspects of the studies may be modified and redefined depending on the circumstance and environment, for example.

### 3.1 Trial A: Normal Patient Population

A patient population, with a normal control is dosed once a day for a week or longer tested. The test is designed to allow for improvement, i.e. that there is a measurable parameter increase of the impaired function. The patients are tested at the beginning and at the end of the dosage period and the results are compared and analyzed. A challenge test may be applied (such as sleep deprivation paradigm) and the potential inclusion of a positive control (e.g. modafinil).

### 3.2 Trial B: Deficit population

A patient population with narcolepsy, EDS, cataplexy and/or nocturnal sleep disruption, is dosed once a day for a week or longer and tested. The test is designed to allow for improvement, i.e. that there is a measurable parameter increase of the impaired domain. Examples of those tests include Epworth sleepiness scale (ESS), a sleep Diary (Number and duration of diurnal sleep and sleepiness episodes, number of cataplexy attacks), Maintenance of Wakefulness Test (MWT), Test of Sustained Attention to Response Task (SART), nocturnal polysomnography and EEG. The patients are tested at the beginning and at the end of the dosage period and the results are compared and analyzed.

### 3.3 Considerations for designing a trial

- When designing a trial, the skilled person will appreciate the need to protect both against floor and ceiling effects. In other words, the study design should allow the specific domain to be measurably raised or lowered.
- Conditions that artificially impair a function, are one way to test enhancement of that function. Such conditions are, for example, sleep deprivation, jet lag or shift work and pharmacological challenges.
- Placebo control is required for all trials.
- A positive control (current approved medication) may applied

### Description of Figures:

Figure 1: Behavioral arrest, compound B-4
Figure 2: Behavioral arrest, compound B-1
Figure 3: EEG Measurements, compound B-1
Figure 4: Telemethyl histamine measurements, compounds B-1 and A-1

The invention also provides a kit comprising an α7 nAChR agonist of the invention and instructions for using the agonist in the treatment, amelioration, prevention or delay of progression of narcolepsy, EDS, nocturnal sleep disruption or cataplexy in a subject in need of such treatment.

The invention also provides a pharmaceutical composition comprising an α7 nAChR agonist of the invention for the treatment, amelioration, prevention or delay of progression of narcolepsy, excessive daytime sleepiness, nocturnal sleep disruption or cataplexy.

## Claims

1. An alpha 7 nicotinic acetylcholine receptor agonist for use in the treatment, amelioration, prevention or delay of progression of narcolepsy, excessive daytime sleepiness, nocturnal sleep disruption or cataplexy;
wherein said alpha 7 nicotinic acetylcholine receptor agonist is (R)-3-(6-p-tolyl-pyridin-3-yloxy)-1-aza-bicyclo[2.2.2]octane in free base form or in acid addition salt form.

2. The alpha 7 nicotinic acetylcholine receptor agonist for use as defined in claim 1, wherein the use is the treatment, amelioration, prevention or delay of progression of narcolepsy.

3. The alpha 7 nicotinic acetylcholine receptor agonist for use as defined in claim 2, wherein the narcolepsy is narcolepsy with cataplexy, narcolepsy without cataplexy or narcolepsy due to medical condition.

4. An alpha 7 nicotinic acetylcholine receptor agonist for use as defined in claim 1 for use in the treatment, amelioration, prevention or delay of progression of excessive daytime sleepiness.

5. The alpha 7 nicotinic acetylcholine receptor agonist for use as defined in claim 1 for use in the treatment, amelioration, prevention or delay of progression of nocturnal sleep disruption.

6. The alpha 7 nicotinic acetylcholine receptor agonist for use as defined in claim 1 for use in the treatment, amelioration, prevention or delay of progression of cataplexy.

7. The alpha 7 nicotinic acetylcholine receptor agonist for use as defined in any of claims 1 to 6, wherein the daily dosage of said agonist is from 1 to 100 mg.

8. The alpha 7 nicotinic acetylcholine receptor agonist for use as defined in claim 3, wherein the narcolepsy is narcolepsy with cataplexy and wherein the daily dosage of said agonist is from 1 to 100 mg.

9. The alpha 7 nicotinic acetylcholine receptor agonist for use as defined in claim 3, wherein the narcolepsy is narcolepsy without cataplexy and wherein the daily dosage of said agonist is from 1 to 100 mg.

10. The alpha 7 nicotinic acetylcholine receptor agonist for use as defined in claim 3, wherein the narcolepsy is narcolepsy due to a medical condition and wherein the daily dosage of said agonist is from 1 to 100 mg.

11. A pharmaceutical composition comprising (R)-3-(6-p-tolyl-pyridin-3-yloxy)-1-aza-bicyclo[2.2.2]octane in free base form or in acid addition salt form, for use in the treatment, amelioration, prevention or delay of progression of narcolepsy, excessive daytime sleepiness, nocturnal sleep disruption or cataplexy.

12. The pharmaceutical composition for use according to claim 11, wherein the amount of (R)-3-(6-p-tolyl-pyridin-3-yloxy)-1-aza-bicyclo[2.2.2]octane is from 1 to 100 mg.

## Patentansprüche

1. Nicotinischer-Alpha-7-Acetylcholinrezeptor-Agonist zur Verwendung bei der Behandlung, Linderung, Vorbeugung oder Verzögerung der Progression von Narkolepsie, übermäßiger Tagesschläfrigkeit, nächtlicher Schlafstörung oder Kataplexie;
wobei es sich bei dem Nicotinischer-Alpha-7-Acetylcholinrezeptor-Agonisten um (R)-3-(6-p-Tolylpyridin-3-yloxy)-1-azabicyclo[2.2.2]octan in freier Basenform oder in Säureadditionssalzform handelt.

2. Nicotinischer-Alpha-7-Acetylcholinrezeptor-Agonist zur Verwendung nach Anspruch 1, wobei es sich bei der Verwendung um die Behandlung, Linderung, Vorbeugung oder Verzögerung der Progression von Narkolepsie handelt.

3. Nicotinischer-Alpha-7-Acetylcholinrezeptor-Agonist zur Verwendung nach Anspruch 2, wobei es sich bei der Narkolepsie um Narkolepsie mit Kataplexie, Narkolepsie ohne Kataplexie oder Narkolepsie aufgrund eines Leidens handelt.

4. Nicotinischer-Alpha-7-Acetylcholinrezeptor-Agonist zur Verwendung nach Anspruch 1 zur Verwendung bei der Behandlung, Linderung, Vorbeugung oder Verzögerung der Progression von übermäßiger Tagesschläfrigkeit.

5. Nicotinischer-Alpha-7-Acetylcholinrezeptor-Agonist zur Verwendung nach Anspruch 1 zur Verwendung bei der Behandlung, Linderung, Vorbeugung oder Verzögerung der Progression von nächtlicher Schlafstörung.

6. Nicotinischer-Alpha-7-Acetylcholinrezeptor-Agonist zur Verwendung nach Anspruch 1 zur Verwendung bei der Behandlung, Linderung, Vorbeugung oder Verzögerung der Progression von Kataplexie.

7. Nicotinischer-Alpha-7-Acetylcholinrezeptor-Agonist zur Verwendung nach einem der Ansprüche 1 bis 6, wobei die Tagesdosis des Agonisten 1 bis 100 mg beträgt.

8. Nicotinischer-Alpha-7-Acetylcholinrezeptor-Agonist zur Verwendung nach Anspruch 3, wobei es sich bei der Narkolepsie um Narkolepsie mit Kataplexie handelt und wobei die Tagesdosis des Agonisten 1 bis 100 mg beträgt.

9. Nicotinischer-Alpha-7-Acetylcholinrezeptor-Agonist zur Verwendung nach Anspruch 3, wobei es sich bei der Narkolepsie um Narkolepsie ohne Kataplexie handelt und wobei die Tagesdosis des Agonisten 1 bis 100 mg beträgt.

10. Nicotinischer-Alpha-7-Acetylcholinrezeptor-Agonist zur Verwendung nach Anspruch 3, wobei es sich bei der Narkolepsie um Narkolepsie aufgrund eines Leidens handelt und wobei die Tagesdosis des Agonisten 1 bis 100 mg beträgt.

11. Pharmazeutische Zusammensetzung, umfassend (R)-3-(6-p-Tolylpyridin-3-yloxy)-1-azabicyclo[2.2.2]-octan in freier Basenform oder in Säureadditionssalzform, zur Verwendung bei der Behandlung, Linderung, Vorbeugung oder Verzögerung der Progression von Narkolepsie, übermäßiger Tagesschläfrigkeit, nächtlicher Schlafstörung oder Kataplexie.

12. Pharmazeutische Zusammensetzung zur Verwendung gemäß Anspruch 11, wobei die Menge an (R)-3-(6-p-Tolylpyridin-3-yloxy)-1-azabicyclo[2.2.2]octan 1 bis 100 mg beträgt.

## Revendications

1. Agoniste de récepteur nicotinique d'acétylcholine alpha 7 pour utilisation dans le traitement, le soulagement, la prévention ou le retard de la progression de la narcolepsie, de la somnolence diurne excessive, d'une perturbation nocturne du sommeil ou de la cataplexie ;
où ledit agoniste de récepteur nicotinique d'acétylcholine alpha 7 est le (R)-3-(6-p-tolyl-pyridin-3-yloxy)-1-aza-bicyclo[2.2.2]octane sous forme de base libre ou sous la forme d'un sel d'addition acide.

2. Agoniste de récepteur nicotinique d'acétylcholine alpha 7 pour utilisation selon la revendication 1, où l'utilisation est le traitement, le soulagement, la prévention ou le retard de progression de la narcolepsie.

3. Agoniste de récepteur nicotinique d'acétylcholine alpha 7 pour utilisation selon la revendication 2, où la narcolepsie est une narcolepsie avec cataplexie, une narcolepsie sans cataplexie ou une narcolepsie due à un état pathologique.

4. Agoniste de récepteur nicotinique d'acétylcholine alpha 7 pour utilisation selon la revendication 1 pour utilisation dans le traitement, le soulagement, la prévention ou le retard de progression de la somnolence diurne excessive.

5. Agoniste de récepteur nicotinique d'acétylcholine alpha 7 pour utilisation selon la revendication 1 pour utilisation dans le traitement, le soulagement, la prévention ou le retard de progression d'une perturbation nocturne du sommeil.

6. Agoniste de récepteur nicotinique d'acétylcholine alpha 7 pour utilisation selon la revendication 1 pour utilisation dans le traitement, le soulagement, la prévention ou le retard de progression de la cataplexie.

7. Agoniste de récepteur nicotinique d'acétylcholine alpha 7 pour utilisation selon l'une quelconque des revendications 1 à 6, où la dose quotidienne dudit agoniste est comprise entre 1 et 100 mg.

8. Agoniste de récepteur nicotinique d'acétylcholine alpha 7 pour utilisation selon la revendication 3, où la narcolepsie est une narcolepsie avec cataplexie et où la dose quotidienne dudit agoniste est comprise entre 1 et 100 mg.

9. Agoniste de récepteur nicotinique d'acétylcholine alpha 7 pour utilisation selon la revendication 3, où la narcolepsie est une narcolepsie sans cataplexie et où la dose quotidienne dudit agoniste est comprise entre 1 et 100 mg.

10. Agoniste de récepteur nicotinique d'acétylcholine alpha 7 pour utilisation selon la revendication 3, où la narcolepsie est une narcolepsie due à un état pathologique et où la dose quotidienne dudit agoniste est comprise entre 1 et 100 mg.

11. Composition pharmaceutique comprenant le (R)-3-(6-p-tolyl-pyridin-3-yloxy)-1-aza-bicyclo[2.2.2]octane sous forme de base libre ou sous forme de sel d'addition acide, pour utilisation dans le traitement, le soulagement, la prévention ou le retard de progression de la narcolepsie, de la somnolence diurne excessive ou de la cataplexie.

12. Composition pharmaceutique pour utilisation selon la revendication 11, où la quantité de (R)-3-(6-p-tolyl-pyridin-3-yloxy)-1-aza-bicyclo[2.2.2]octane est comprise entre 1 et 100 mg.
